# EUROPEAN PATENT APPLICATION

(11) **EP 4 427 729 A1**
(43) Date of publication of application: **11.09.2024**
(21) Application number: 22890218.5
(22) Date of filing: 17.10.2022
(51) Int. Cl.: A61K 8/34, A61K 8/37, A61Q 19/00

(54) **PRESERVATIVE FOR EXTERNAL SKIN APPLICATION COMPRISING MAGNOLOL, HONOKIOL, OR COMBINATION THEREOF, AND COSMETIC COMPOSITION COMPRISING SAME**

(30) Priority: 05.11.2021 KR 20210151182
(71) Applicant: Activon Co., Ltd., Cheongju-si, Chungcheongbuk-do 28104 (KR)
(72) Inventor: KIM, Myo Deok, Cheongju-si, Chungcheongbuk-do 28113 (KR); CHO, Se Hee, Seoul 07691 (KR); CHO, Myung Chan, Seoul 06310 (KR); JUNG, Sung Won, Yongin-si, Gyeonggi-do 16909 (KR); HA, Wang Su, Suwon-si, Gyeonggi-do 16683 (KR); KIM, Ha Na, Suwon-si, Gyeonggi-do 16681 (KR); CHO, Youn Ki, Yongin-si, Gyeonggi-do 17005 (KR)
(74) Representative: Tiburzi, Andrea
(86) International application number: PCT/KR2022/015689
(87) International publication number: WO 2023/080483

(57) **Abstract**

The present application relates to a preservative comprising magnolol, honokiol, or a combination thereof and use thereof, and provides a preservative for external skin application that exhibits excellent antibacterial or antiseptic effects on bacteria and fungi by addressing the issue of insolubility of magnolol and honokiol, and a cosmetic composition comprising same.

## Description

### Technical Field

The present disclosure relates to a preservative for external skin application including magnolol, honokiol, or a combination thereof, and a cosmetic composition including the same. This application claims the benefit of Korean Patent Application No. 10-2021-0151182, filed on November 5, 2021, in the Korean Intellectual Property Office, the disclosure of which is incorporated herein in its entirety by reference.

### Background Art

A preservative improving preservation effect of products is essentially required in the manufacture of cosmetic products, because contamination caused by microorganisms cannot be avoided during processes of manufacturing and using the cosmetic products. As such preservatives, paraben preservatives, imidazolidinyl urea, phenoxyethanol, and the like are widely used. However, despite the superior merits of an excellent preservation effect due to high antibacterial activity, drawbacks of the above-described preservatives, such as toxicity, skin irritation, and allergy, are also widely known. Therefore, there is a need to develop a safe preservative effect with excellent preservation ability without causing skin irritation.

Meanwhile, magnolol and honokiol, obtained from stems of magnolias, have been reported to have anxiety relief effects, anticancer effects, and antiplatelet effects, and are also known to be used for increasing skin volume and relieving wrinkles (Korean Patent No. 10-2046331). However, magnolol and honokiol are not commercially used at all due to the issue of complete insolubility in water or oil. Precipitation occurs over time even in emulsions or other suspensions using a sufficient amount of a surfactant or solvent at a concentration of about 0.1% or more, and precipitation also occurs within a short period of time even in a solubilized liquid formulation (medicines, cosmetics, food, household goods, disinfectants, pesticides, and the like). Once precipitation occurs, activity of magnolol and honokiol is lost in any case. Therefore, if it is possible to manufacture a product by dissolving these two substances in water or oil by reducing insolubility thereof without losing the activity thereof, the substances may be sufficiently commercially available, but this has still not been achieved.

### Disclosure

### Technical Problem

To solve these issues, the present inventors have found a preservative for external skin application including magnolol, honokiol, or a combination thereof, wherein because the preservative for external skin application has excellent formulation stability capable of maintaining a stable transparent, liquid state without precipitation and solidification, the preservative may solve the issue of insolubility of magnolol and honokiol and may be stably applied to a cosmetic composition to provide excellent antibacterial or antiseptic effects, thereby completing the present disclosure.

Provided is a preservative for external skin application including at least one compound selected from the group consisting of magnolol and honokiol; and at least one solvent selected from the group consisting of heptyl undecylenate, propanediol, butyleneglycol, ethylhexylglycerin, and glyceryl caprylate.

Provided is a cosmetic composition including the preservative for external skin application.

Provided is a method of preparing a preservative for external skin application including at least one compound selected from the group consisting of magnolol and honokiol; and at least one solvent selected from the group consisting of heptyl undecylenate, propanediol, butyleneglycol, ethylhexylglycerin, and glyceryl caprylate.

Provided is a method of preparing a cosmetic composition, the method including adding the preservative for external skin application.

Provided is a method of applying antibacterial, preservative, or antiseptic treatment to a cosmetic composition, the method including adding the preservative for external skin application.

Provided is a use of a composition including at least one compound selected from the group consisting of magnolol and honokiol; and at least one solvent selected from the group consisting of heptyl undecylenate, propanediol, butyleneglycol, ethylhexylglycerin, and glyceryl caprylate, for antibacterial, preservative, or antiseptic treatment of an agent for external skin application or a cosmetic composition.

Provided is a use of a composition including at least one compound selected from the group consisting of magnolol and honokiol; and at least one solvent selected from the group consisting of heptyl undecylenate, propanediol, butyleneglycol, ethylhexylglycerin, and glyceryl caprylate, for preparing a preservative for external skin application or an antibacterial or antiseptic cosmetic composition.

However, technical problems to be solve in the present disclosure are not limited to the above-described problems, and other problems which are not described herein will be fully understood by those of ordinary skill in the art from the following descriptions.

### Technical Solution

Unless otherwise defined, all technical and scientific terms used herein have the same meaning as commonly understood by one of ordinary skill in the art to which the present disclosure belongs. Generally, the nomenclature used herein and the experiment methods, which will be described below, are those well known and commonly employed in the art.

According to an aspect of the present disclosure, a preservative for external skin application includes at least one compound selected from the group consisting of magnolol and honokiol; and at least one solvent selected from the group consisting of heptyl undecylenate, propanediol, butyleneglycol, ethylhexylglycerin, and glyceryl caprylate.

As used herein, the term "magnolol" is an organic compound classified as lignin referring to a biologically active compound found in back of Magnolia officinalis or M. grandiflora. Specifically, the magnolol may be a compound having a structure of Formula 1 below:

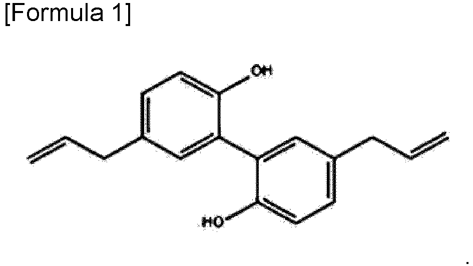

The magnolol may have a molecular formula of C₁₈H₁₈O₂ and a molecular weight of about 260 to 270 g/mol.

As used herein, the term "honokiol" is an organic compound classified as lignin referring to a biologically active compound found in barks, seeds, and leaves of trees belonging to magnolia. Specifically, the honokiol may be a compound having a structure of Formula 2 below:

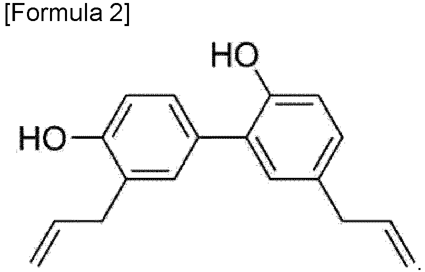

The honokiol may have a molecular formula of C₁₈H₁₈O₂ and a molecular weight of 260 to 270 g/mol.

The preservative for external skin application may include the at least one compound selected from the group consisting of magnolol and honokiol in an amount of more than about 0.5 but less than 12 wt% based on a total weight. Specifically, the preservative for external skin application may include the at least one compound selected from the group consisting of magnolol and honokiol in an amount of about 1 or more but less than 12, 1 to 11, 1 to 10, 1 to 7, 1 to 5, 1 to 3, 3 or more but less than 12, 3 to 11, 3 to 10, 3 to 7, 3 to 5, 5 or more but less than 12, 5 to 11, 5 to 10, 5 to 7, 7 or more but less than 12, 7 to 11, or 7 to 10 wt% based on the total weight.

According to an embodiment, if the preservative for external skin application includes the at least one compound selected from the group consisting of magnolol and honokiol in an amount of about 0.5 wt% or less based on the total weight, antibacterial or antiseptic effects may decrease. If the amount is about 12 wt% or more, deterioration in stability, such as occurrence of solidification or precipitation, decrease in solubility, or reduction in emulsifying or solubilizing activity may be caused.

The preservative for external skin application may include the at least one solvent selected from the group consisting of heptyl undecylenate, propanediol, butyleneglycol, ethylhexylglycerin, and glyceryl caprylate in an amount of more than about 88 but less than 99.5 wt% based on the total weight. Specifically, the preservative for external skin application may include the at least one solvent selected from the group consisting of heptyl undecylenate, propanediol, butyleneglycol, ethylhexylglycerin, and glyceryl caprylate in an amount of about 89 to 99, 90 to 99, 93 to 99, 95 to 99, 97 to 99, 89 to 97, 90 to 97, 93 to 97, 95 to 97, 89 to 95, 90 to 95, 93 to 95, 89 to 93, 90 to 93, 89 to 90, more than 88 but not more than 97, more than 88 but not more than 95, 95, or 90 wt% based on the total weight. According to an embodiment, if the preservative for external skin application includes the at least one solvent selected from the group consisting of heptyl undecylenate, propanediol, butyleneglycol, ethylhexylglycerin, and glyceryl caprylate in an amount of about 88 wt% or less based on the total weight, the amount of the at least one compound selected from the group consisting of magnolol and honokiol may inevitably increase, thereby causing deterioration in stability, such as occurrence of solidification or precipitation, decrease in solubility, or reduction in emulsifying or solubilizing activity. If the amount is about 99.5 wt% or more, the amount of the at least one compound selected from the group consisting of magnolol and honokiol may inevitably decrease, resulting in a decrease in antibacterial or antiseptic effects.

The preservative for external skin application may include about 1 to 10, 5 to 10, 3 or more but less than 12, 3 to 11, 5 or more but less than 12, 5 to 11, 10, or 5 wt% of a total amount of the at least one compound selected from the group consisting of magnolol and honokiol; and about 90 to 99, 90 to 95, 89 to 97, 89 to 95, more than 88 but not more then 97, more than 88 but not more than 95, 95, or 90 wt% of a total amount of the at least one solvent selected from the group consisting of heptyl undecylenate, propanediol, butyleneglycol, ethylhexylglycerin, and glyceryl caprylate, based on the total weight. Alternatively, the preservative for external skin application may include about 1 to 10, 5 to 10, 3 or more but less than 12, 3 to 11, 5 or more but less than 12, 5 to 11, 10, or 5 parts by weight of a total weight of the at least one compound selected from the group consisting of magnolol and honokiol; and about 90 to 99, 90 to 95, 89 to 97, 89 to 95, more than 88 but not more than 97, more than 88 but not more than 95, 95, or 90 parts by weight of a total amount of the at least one solvent selected from the group consisting of heptyl undecylenate, propanediol, butyleneglycol, ethylhexylglycerin, and glyceryl caprylate.

According to an embodiment, if the amounts or ratio of the total amount of the at least one compound selected from the group consisting of magnolol and honokiol; and the total amount of the at least one solvent selected from the group consisting of heptyl undecylenate, propanediol, butyleneglycol, ethylhexylglycerin, and glyceryl caprylate, which are included in the preservative for external skin application, are out of the ranges of wt% or parts by weight, antibacterial or antiseptic effects of the preservative for external skin application may significantly decrease or solidification or precipitation, decrease in solubility, or reduction in emulsifying or solubilizing activity may occur, resulting in significant deterioration in stability of the preservative for external skin application.

A weight ratio of the total amount of the at least one compound selected from the group consisting of magnolol and honokiol to the total amount of the at least one solvent selected from the group consisting of heptyl undecylenate, propanediol, butyleneglycol, ethylhexylglycerin, and glyceryl caprylate contained in the preservative for external skin application may be about 1 to 10:90 to 99. Specifically, the weight ratio of the total amount of the at least one compound selected from the group consisting of magnolol and honokiol to the total amount of the at least one solvent selected from the group consisting of heptyl undecylenate, propanediol, butyleneglycol, ethylhexylglycerin, and glyceryl caprylate may be about 5 to 10:90 to 95; 1:8 to 150; 1:9 to 100; 1:9 to 99; 1:9; or 1:19. According to an embodiment, if the weight ratio of the total amount of the at least one compound selected from the group consisting of magnolol and honokiol to the total amount of the at least one solvent selected from the group consisting of heptyl undecylenate, propanediol, butyleneglycol, ethylhexylglycerin, and glyceryl caprylate, which are contained in the preservative for external skin application, is out of the above weight ratio ranges, antibacterial or antiseptic effects of the preservative for external skin application may significantly decrease, or solidification or precipitation, decrease in solubility, or reduction in emulsifying or solubilizing activity may occur, resulting in significant deterioration in stability of the preservative for external skin application.

The preservative for external skin application may include: magnolol; honokiol; and at least one solvent selected from the group consisting of heptyl undecylenate, propanediol, butyleneglycol, ethylhexylglycerin, and glyceryl caprylate.

The preservative for external skin application may include the magnolol in an amount more than about 0.25 but less than 6 wt% based on the total weight. Specifically, the preservative for external skin application may include the magnolol in an amount of about 0.5 or more but less than 6, 0.5 to 5.5, 0.5 to 5, 0.5 to 4.5, 0.5 to 4, 0.5 to 3.5, 0.5 to 3, 0.5 to 2.5, 0.5 to 2, 0.5 to 1.5, 0.5 to 1, 1 or more but less than 6, 1 to 5.5, 1 to 5, 1 to 4.5, 1 to 4, 1 to 3.5, 1 to 3, 1 to 2.5, 1 to 2, 1 to 1.5, 1.5 or more but less than 6, 1.5 to 5.5, 1.5 to 5, 1.5 to 4.5, 1.5 to 4, 1.5 to 3.5, 1.5 to 3, 1.5 to 2.5, 1.5 to 2, 2 or more but less than 6, 2 to 5.5, 2 to 5, 2 to 4.5, 2 to 4, 2 to 3.5, 2 to 3, 2 to 2.5, 2.5 or more but less than 6, 2.5 to 5.5, 2.5 to 5, 2.5 to 4.5, 2.5 to 4, 2.5 to 3.5, 2.5 to 3, 3 or more but less than 6, 3 to 5.5, 3 to 5, 3 to 4.5, 3 to 4, 3 to 3.5, 3.5 or more but less than 6, 3.5 to 5.5, 3.5 to 5, 3.5 to 4.5, 3.5 to 4, 4 or more but less than 6, 4 to 5.5, 4 to 5, 4 to 4.5, 4.5 or more but less than 6, 4.5 to 5.5, 4.5 to 5, 5 or more but less than 6, 5 to 5.5, 5.5 or more but less than 6, 5, or 2.5 wt% based on the total weight. According to an embodiment, if the preservative for external skin application includes the magnolol in an amount of about 0.25 wt% or less based on the total weight, antibacterial or antiseptic effects may decrease. If the amount is about 6 wt% or more, deterioration in stability, such as occurrence of solidification or precipitation, decrease in solubility, or reduction in emulsifying or solubilizing activity may be caused.

The preservative for external skin application may include the honokiol in an amount of about more than 0.25 but less than 6 wt% based on the total weight. Specifically, the preservative for external skin application may include the honokiol in an amount of about 0.5 or more but less than 6, 0.5 to 5.5, 0.5 to 5, 0.5 to 4.5, 0.5 to 4, 0.5 to 3.5, 0.5 to 3, 0.5 to 2.5, 0.5 to 2, 0.5 to 1.5, 0.5 to 1, 1 or more but less than 6, 1 to 5.5, 1 to 5, 1 to 4.5, 1 to 4, 1 to 3.5, 1 to 3, 1 to 2.5, 1 to 2, 1 to 1.5, 1.5 or more but less than 6, 1.5 to 5.5, 1.5 to 5, 1.5 to 4.5, 1.5 to 4, 1.5 to 3.5, 1.5 to 3, 1.5 to 2.5, 1.5 to 2, 2 or more but less than 6, 2 to 5.5, 2 to 5, 2 to 4.5, 2 to 4, 2 to 3.5, 2 to 3, 2 to 2.5, 2.5 or more but less than 6, 2.5 to 5.5, 2.5 to 5, 2.5 to 4.5, 2.5 to 4, 2.5 to 3.5, 2.5 to 3, 3 or more but less than 6, 3 to 5.5, 3 to 5, 3 to 4.5, 3 to 4, 3 to 3.5, 3.5 or more but less than 6, 3.5 to 5.5, 3.5 to 5, 3.5 to 4.5, 3.5 to 4, 4 or more but less than 6, 4 to 5.5, 4 to 5, 4 to 4.5, 4.5 or more but less than 6, 4.5 to 5.5, 4.5 to 5, 5 or more but less than 6, 5 to 5.5, 5.5 or more but less than 6, 5, or 2.5 wt% based on the total weight. According to an embodiment, if the preservative for external skin application includes the honokiol in an amount of about 0.25 wt% or less based on the total weight, antibacterial or antiseptic effects may decrease. If the amount is about 6 wt% or more, deterioration in stability, such as occurrence of solidification or precipitation, decrease in solubility, or reduction in emulsifying or solubilizing activity may be caused.

The preservative for external skin application may include about 0.5 to 5, 2.5 to 5, 1.5 or more but less than to 6, 1.5 to 5.5, 2.5 or more but less than 6, 2.5 to 5.5, 5, or 2.5 wt% of magnolol; about 0.5 to 5, 2.5 to 5, 1.5 or more but less than 6, 1.5 to 5.5, 2.5 or more but less than 6, 2.5 to 5.5, 5, or 2.5 wt% of honokiol; and about 90 to 99, 90 to 95, 89 to 97, 89 to 95, more than 88 but not more than 97, more than 88 but not more than 95, 95, or 90 wt% of the at least one solvent selected from the group consisting of heptyl undecylenate, propanediol, butyleneglycol, ethylhexylglycerin, and glyceryl caprylate, based on the total weight. Alternatively, the preservative for external skin application may include about 0.5 to 5, 2.5 to 5, 1.5 or more but less than 6, 1.5 to 5.5, 2.5 or more but less than 6, 2.5 to 5.5, 5, or 2.5 parts by weight of magnolol; about 0.5 to 5, 2.5 to 5, 1.5 or more but less than 6, 1.5 to 5.5, 2.5 or more but less than 6, 2.5 to 5.5, 5, or 2.5 parts by weight of honokiol; and about 90 to 99, 90 to 95, 89 to 97, 89 to 95, more than 88 but not more than 97, more than 88 but not more than 95, 95, or 90 parts by weight of the at least one solvent selected from the group consisting of heptyl undecylenate, propanediol, butyleneglycol, ethylhexylglycerin, and glyceryl caprylate.

According to an embodiment, if the amounts or ratio of the magnolol; the honokiol; and the total amount of the at least one solvent selected from the group consisting of heptyl undecylenate, propanediol, butyleneglycol, ethylhexylglycerin, and glyceryl caprylate, included in the preservative for external skin application, is out of the wt% or parts by weight, antibacterial or antiseptic effects of the preservative for external skin application may significantly decrease, or solidification or precipitation, decrease in solubility, or reduction in emulsifying or solubilizing activity may occur, resulting in significant deterioration in stability of the preservative for external skin application.

A weight ratio of the magnolol; the honokiol; and the total amount of the at least one solvent selected from the group consisting of heptyl undecylenate, propanediol, butyleneglycol, ethylhexylglycerin, and glyceryl caprylate, included in the preservative for external skin application, may be 0.5 to 5:0.5 to 5:90 to 99. Specifically, the weight ratio of the magnolol; the honokiol; and the total amount of the at least one solvent selected from the group consisting of heptyl undecylenate, propanediol, butyleneglycol, ethylhexylglycerin, and glyceryl caprylate may be about 2.5 to 5:2.5 to 5:90 to 95; 1:1:15 to 250; 1:1:18 to 200; 1:1:18to 198; 1:1:18; or 1:1:38. According to an embodiment, if the weight ratio of the magnolol; the honokiol; and the total amount of the at least one solvent selected from the group consisting of heptyl undecylenate, propanediol, butyleneglycol, ethylhexylglycerin, and glyceryl caprylate, included in the preservative for external skin application, is out of the weight ratio range, antibacterial or antiseptic effects of the preservative for external skin application may significantly decrease, or solidification or precipitation, decrease in solubility, or reduction in emulsifying or solubilizing activity may occur, resulting in significant deterioration in stability of the preservative for external skin application.

The preservative for external skin application may include magnolol; honokiol; and heptyl undecylenate.

The preservative for external skin application may include about 0.5 to 5, 2.5 to 5, 1.5 or more but less than 6, 1.5 to 5.5, 2.5 or more but less than 6, 2.5 to 5.5, 5, or 2.5 wt% of magnolol; about 0.5 to 5, 2.5 to 5, 1.5 or more but less than 6, 1.5 to 5.5, 2.5 or more but less than 6, 2.5 to 5.5, 5, or 2.5 wt% of honokiol; and about 90 to 99, 90 to 95, 89 to 97, 89 to 95, more than 88 but not more than 97, more than 88 but not more than 95, 95, or 90 wt% of heptyl undecylenate, based on the total weight. Alternatively, the preservative for external skin application may include about 0.5 to 5, 2.5 to 5, 1.5 or more but less than 6, 1.5 to 5.5, 2.5 or more but less than 6, 2.5 to 5.5, 5, or 2.5 parts by weight of magnolol; about 0.5 to 5, 2.5 to 5, 1.5 or more but less than 6, 1.5 to 5.5, 2.5 or more but less than 6, 2.5 to 5.5, 5, or 2.5 parts by weight of honokiol; and about 90 to 99, 90 to 95, 89 to 97, 89 to 95, more than 88 but not more than 97, more than 88 but not more than 95, 95, or 90 parts by weight of heptyl undecylenate.

According to an embodiment, if the amounts or ratio of magnolol; honokiol; and heptyl undecylenate included in the preservative for external skin application are out of the range of wt% or parts by weight, antibacterial or antiseptic effects of the preservative for external skin application may significantly decrease, or solidification or precipitation, decrease in solubility, or reduction in emulsifying or solubilizing activity may occur, resulting in significant deterioration in stability of the preservative for external skin application.

The weight ratio of the magnolol; the honokiol; and the heptyl undecylenate included in the preservative for external skin application may be 0.5 to 5:0.5 to 5:90 to 99, 2.5 to 5:2.5 to 5:90 to 95; 1:1:15 to 250; 1:1:18 to 200; 1:1:18 to 198; 1:1:18; or 1:1:38. According to an embodiment, if the weight ratio of the magnolol; the honokiol; and the heptyl undecylenate included in the preservative for external skin application is out of the weight ratio range, antibacterial or antiseptic effects of the preservative for external skin application may significantly decrease, or solidification or precipitation, decrease in solubility, or reduction in emulsifying or solubilizing activity may occur, resulting in significant deterioration in stability of the preservative for external skin application.

According to an embodiment, due to excellent antibacterial activity, antiseptic activity, and safety, the preservative for external skin application may be used as a preservative, an antiseptic agent, or an antiseptic adjuvant safe for skin in the case of being applied to a cosmetic composition.

In addition, according to an embodiment, if the preservative for external skin application includes at least one compound selected from the group consisting of magnolol and honokiol; and at least one solvent selected from the group consisting of heptyl undecylenate, propanediol, butyleneglycol, ethylhexylglycerin, and glyceryl caprylate, the preservative has excellent formulation stability capable of maintaining a stable, transparent liquid state without causing discoloration, off-flavor, precipitation, and solidification under various temperature conditions, specifically, under room temperature and refrigeration conditions, thereby solving the solidification or precipitation phenomenon, i.e., insolubility issue of magnolol and honokiol, that may be caused in the case of mixing magnolol, honokiol, or a combination thereof with a solvent. Therefore, the preservative for external skin application may be stably applied to cosmetic compositions to exhibit excellent antibacterial or antiseptic effects.

In addition, according to an embodiment, the preservative for external skin application may have excellent antibacterial or antiseptic effects on various types of microorganisms (specifically, contaminating microorganisms). Specifically, the preservative for external skin application may suppress the growth of or kill gram-positive bacteria and fungi (e.g., yeast and mold). Specifically, the preservative for external skin application may have excellent antibacterial or antiseptic effects on Staphylococcus aureus, Cutibacterium acnes, Candida albicans, Malassezia furfur, Trichophyton rubrum, and Aspergillus brasiliensis, and the like, without being limited thereto.

In addition, according to an embodiment, the preservative for external skin application has excellent safety for external skin application due to significantly low skin irritation, compared to use of preservatives commonly used in conventional cosmetic products (such as paraben preservatives, imidazolidinyl urea, and phenoxyethanol), octanediol, caprylylglycerylether, or ethylhexylglycerin alone.

As used herein, the term "agent for external skin application" is a concept including compositions applied to skin, for example, a concept including various cosmetics such as basic cosmetics, makeup cosmetics, and hair cosmetics; and pharmaceutical compositions for topical administration including medicines and quasi-drug products such as ointments, creams, and lotions.

As used herein, the term "preservative for external skin application" may be used interchangeably with "antibacterial agent", "antiseptic agent ", "antibacterial preservative", "antiseptic preservative", "antibacterial composition", "antiseptic composition", "antibacterial or antiseptic composition", "antibacterial cosmetic composition", "antiseptic cosmetic composition", and "antibacterial or antiseptic cosmetic composition".

As used herein, the term "antibacterial" refers to resistant to all contaminating microorganisms such as bacteria, mold, and yeast, the "antibacterial activity" or "antibacterial effect" refers to defensive ability or defensive effect on the contaminating microorganisms, and the "antiseptic activity" or "antiseptic effect" refers to defensive ability or defensive effect on decomposition caused by contamination of microorganisms as a concept including antibacterial activity or antibacterial effect.

As used herein, the term "preservative" refers to a substance having antiseptic activity or antioxidant properties capable of inhibiting denaturation of a product and maintaining the original state of the product for a long period of time, and the "preservation ability" refers to a defensive ability to prevent denaturation of a product and maintain the original state thereof for a long time.

The preservative for external skin application may further include an additive such as purified water, a carrier, an emulsifier, a moisturizer, a skin conditioner, a surfactant, a chelating agent, an antioxidant, an antiseptic agent, a disinfectant, a stabilizer, a preservative, a pH regulator, a lubricant, a solubilizer, and a solvent. In addition, the preservative for external skin application may further include a substance capable of additionally providing essential nutrients, a substance capable of improving the condition of skin, e.g., a functional substance having effects on reducing wrinkles or improving whitening. Also, the preservative for external skin application may further include an adjuvant such as natural fragrance, cosmetic fragrance, or plant extract without being limited thereto.

According to another aspect of the present disclosure, a cosmetic composition includes the preservative for external skin application.

The cosmetic composition may include the preservative for external skin application in an amount of about 0.0001 to 50 wt% based on a total weight of the cosmetic composition. Specifically, the cosmetic composition may include the preservative for external skin application in an amount of about 0.0001 to 0.001, 0.001 to 0.01, 0.01 to 0.1, 0.1 to 1, 1 to 10, 10 to 20, 20 to 30, 30 to 40, or 40 to 50 wt% based on the total weight of the cosmetic composition. According to an embodiment, if the cosmetic composition includes the preservative for external skin application in an amount less than about 0.0001 wt% based on the total weight of the cosmetic composition, antibacterial or antiseptic effects may decrease. If the cosmetic composition includes the preservative for external skin application in an amount more than about 50 wt% based on the total weight of the cosmetic composition, deterioration in stability such as occurrence of solidification or precipitation, decrease in solubility, or reduction in emulsifying or solubilizing activity may be caused.

The cosmetic composition may be a composition of cosmetic raw materials used to manufacture cosmetics. In addition, the cosmetic composition may be a cosmetic composition having a final cosmetic formulation.

The cosmetic composition may further include an additive selected from the group consisting of purified water, carriers, emulsifiers, moisturizers, skin conditioners, surfactants, chelating agents, antioxidants, antiseptic agents, disinfectants, stabilizers, preservatives, pH regulators, lubricants, solubilizers, solvents, and combinations thereof, without being limited thereto. In addition, the cosmetic composition may further include a substance capable of additionally providing essential nutrients, a substance capable of improving skin condition, e.g., a functional substance having effects on reducing wrinkles or improving whitening. The cosmetic composition may further include an adjuvant such as natural fragrance, cosmetic fragrance, or plant extract without being limited thereto.

The cosmetic composition may be prepared in any formation commonly prepared in the art. Specifically, the cosmetic composition may be in a formulation such as solution, emulsion, suspension, softening lotion, nourishing lotion, massage cream, nourishing cream, pack, gel, skin-adhesive cosmetics, lipstick, powder, makeup base, foundation, shampoo, rinse, scalp cleaner, scalp lotion, scalp cream, scalp pack, scalp gel, scalp ointment, scalp gel, body cleanser, soap, toothpaste, mouthwash, paste, lotion, gel, cream, patch, spry, or aerosol, without being limited thereto.

The carrier included in the cosmetic composition may be selectively used depending on the formation of cosmetics. For example, when cosmetics in a formulation of ointment, paste, cream, or gel are manufactured, wax, paraffin, starch, tragacanth, cellulose derivative, polyethylene glycol, silicone, bentonites, silica, talc, zinc oxide, and the like may be used alone or in combination as a carrier component. When cosmetics in a formulation of powder or spray are manufactured, lactose, talc, silica, aluminum hydroxide, calcium silicate, polyamide powder, chlorofluorohydrocarbon, propane/butane, dimethyl ether and the like may be used alone or in combination as a carrier component. When cosmetics in a formulation of solution or emulsion are manufactured, water, ethanol, isopropanol, ethyl carbonate, ethyl acetate, benzyl alcohol, benzyl benzoate, propylene glycol, 1,3-butylglycol oil, cottonseed oil, peanut oil, corn germ oil, olive oil, caster oil, sesame oil, glycerol aliphatic ester, polyethylene glycol, sorbitan and the like may be used alone or in combination as a carrier component. When cosmetics in a formulation of suspension are manufactured, water, ethanol, propylene glycol, ethoxylated isostearyl alcohol, polyoxyethylene sorbitol ester, and polyoxyethylene sorbitan ester; microcrystalline cellulose, aluminum meta-hydroxide, bentonite, agar, tragacanth, and the like may be used alone or in combination as a carrier component. When cosmetics in a formulation of soap are manufactured, alkali metal salt of fatty acid, hemiester salt of fatty acid, fatty acid protein hydrolysate, isethionate, lanolin derivative, aliphatic alcohol, vegitable oil, glycerl, saccharide, and the like may be used alone or in combination as a carrier component.

Among the terms or components described in the cosmetic compositions, those given in the descriptions of the preservative for external skin application are understood as mentioned above in the descriptions of the preservative for external skin application.

According to another aspect of the present disclosure, a method of preparing a preservative for external skin application includes mixing at least one compound selected from the group consisting of magnolol and honokiol; and at least one solvent selected from the group consisting of heptyl undecylenate, propanediol, butyleneglycol, ethylhexylglycerin, and glyceryl caprylate.

According to another aspect of the present disclosure, a method of preparing a cosmetic composition includes adding the preservative for external skin application.

According to another aspect of the present disclosure, a method of performing antibacterial, preservative, or antiseptic treatment on a cosmetic composition includes adding the preservative for external skin application.

In the methods, the mixing weight ratio, wt%, or parts by weight of the components are as described above in the preservative for external skin application.

Among the terms or components described in the methods, those given in the descriptions of the preservative for external skin application or the cosmetic composition are understood as mentioned above in the descriptions of the preservative for external skin application or the cosmetic composition.

According to another aspect of the present disclosure, a use of a composition including at least one compound selected from the group consisting of magnolol and honokiol; and at least one solvent selected from the group consisting of heptyl undecylenate, propanediol, butyleneglycol, ethylhexylglycerin, and glyceryl caprylate is for antibacterial, preservative, or antiseptic treatment of an agent for external skin application or a cosmetic composition.

According to another aspect of the present disclosure, a use of a composition including at least one compound selected from the group consisting of magnolol and honokiol; and at least one solvent selected from the group consisting of heptyl undecylenate, propanediol, butyleneglycol, ethylhexylglycerin, and glyceryl caprylate is for preparing a preservative for external skin application or an antibacterial or antiseptic cosmetic composition.

The mixing weight ratio, wt%, or parts by weight of the components of the composition including the at least one compound selected from the group consisting of magnolol and honokiol; and at least one solvent selected from the group consisting of heptyl undecylenate, propanediol, butyleneglycol, ethylhexylglycerin, and glyceryl caprylate are as described above in the preservative for external skin application.

Among the terms or components described in the uses, those given in the descriptions of the preservative for external skin application, the cosmetic composition, or the methods are understood as mentioned above in the descriptions of the preservative for external skin application, the cosmetic composition, or the methods.

### Advantageous Effects

Due to excellent antibacterial activity, antiseptic activity, and safety, the preservative for external skin application including magnolol, honokiol, or a combination thereof according to an embodiment may be used as a preservative, an antiseptic agent, or an antiseptic adjuvant safe for skin when applied to a cosmetic composition.

Also, because the preservative for external skin application has excellent formulation stability capable of maintaining a stable, transparent liquid state without causing discoloration, off-flavor, precipitation, and solidification under various temperature conditions, specifically, under room temperature and refrigeration conditions, the solidification or precipitation phenomenon, i.e., insolubility issue, of magnolol and honokiol, which may be caused in the case of mixing magnolol, honokiol, or a combination thereof with a solvent may be solved. Therefore, the preservative for external skin application including magnolol, honokiol, or a combination thereof may be stably applied to cosmetic compositions and exhibit excellent antibacterial or antiseptic effects.

### Description of Drawings

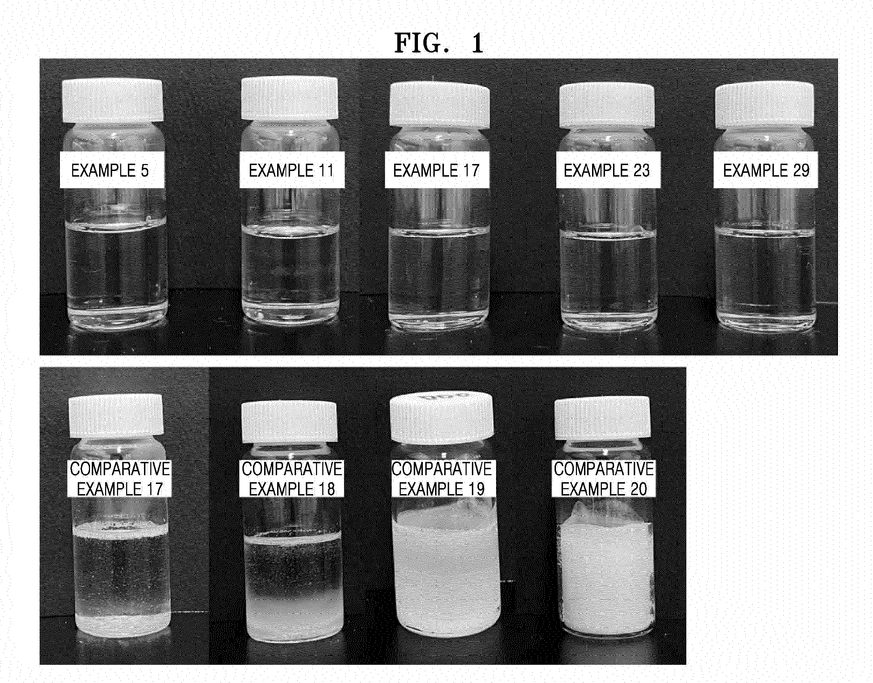

FIG. 1 is images showing results of identifying formulation stability after storing preservatives for external skin application of Examples 5, 11, 17, 23, and 29 including magnolol; honokiol; and heptyl undecylenate, propanediol, butyleneglycol, ethylhexylglycerin, or glyceryl caprylate, and preservatives for external skin application of Comparative Examples 17 to 20 including magnolol; honokiol; and pentylene glycol, hexanediol, carprylyl glycol, or decanediol, under room temperature conditions (at about 25 °C).

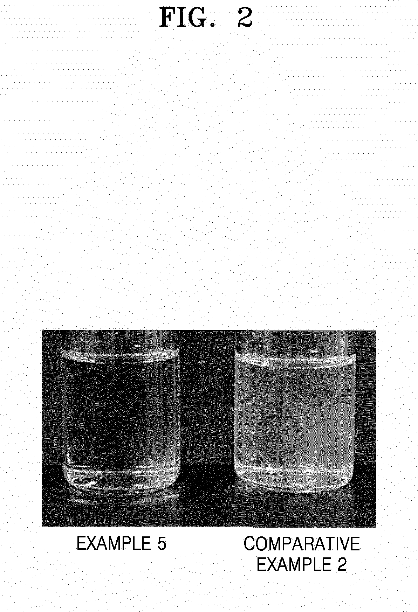

FIG. 2 is an image showing results of identifying formulation stability after storing the preservative for external skin application of Example 5 including magnolol; honokiol; and heptyl undecylenate, in a weight ratio of 5:5:90, and a preservative for external skin application of Comparative Example 2 including magnolol; honokiol; and heptyl undecylenate, in a weight ratio of 6:6:88, under room temperature conditions (at about 25 °C).

### Mode for Invention

Hereinafter, the present disclosure will be described in more detail with reference to the following examples. However, the following examples are merely presented by exemplify the present disclosure, and the scope of the present disclosure is not limited thereto.

### 1. Preparation and Evaluation of Preservative for External Skin Application

### Example 1 - Example 3. Preparation of Preservative for External Skin Application

In these examples, preservatives for external skin application including magnolol, honokiol, or a combination thereof were prepared.

Specifically, magnolol, honokiol, or a combination thereof; and a solvent were mixed at room temperature and stirred to prepare a preservative for external skin application, and then 53 types of preservatives for external skin application (Examples 1 to 33 and Comparative Examples 1 to 20) were obtained by varying weight ratio (wt% of the mixed raw materials and types of the solvent. The results are shown in Tables 1 to 5 below. As the solvent, one selected from heptyl undecylenate, propanediol, butyleneglycol, ethylhexylglycerin, glyceryl caprylate, pentylene glycol, hexanediol, carprylyl glycol, and decanediol was used.

**[Table 1]**

| Raw material (unit: wt%) | Exa mpl e 1 | Exa mpl e 2 | Exa mple 3 | Exa mpl e 4 | Exa mpl e 5 | Exa mpl e 6 | Exa mpl e 7 | Ex am ple 8 | Ex am ple 9 | Exa mpl e 10 | Ex am ple 11 | Ex am ple 12 | Exa mpl e 13 | Exa mple 14 | Exa mpl e 15 |
|---|---|---|---|---|---|---|---|---|---|---|---|---|---|---|---|
| magnolol | 0.5 | 1 | - | 2.5 | 5 | 5 | 10 | - | - | 0.5 | 5 | 5 | 10 | - | - |
| honokiol | 0.5 | - | 1 | 2.5 | 5 | - | - | 5 | 10 | 0.5 | 5 | - | - | 5 | 10 |
| heptyl undecylenate | 99 | 99 | 99 | 95 | 90 | 95 | 90 | 95 | 90 | - | - | - | - | - | - |
| propanediol | - | - | - | - | - | - | - | - | - | 99 | 90 | 95 | 90 | 95 | 90 |

**[Table 2]**

| Raw material (unit: wt%) | Exa mple 16 | Exa mple 17 | Exa mple 18 | Exa mple 19 | Exa mple 20 | Exa mple 21 | Exa mple 22 | Exa mple 23 | Exa mple 24 | Exa mple 25 | Exa mple 26 | Exa mpl e 27 |
|---|---|---|---|---|---|---|---|---|---|---|---|---|
| magnolol | 0.5 | 5 | 5 | 10 | - | - | 0.5 | 5 | 5 | 10 | - | - |
| honokiol | 0.5 | 5 | - | - | 5 | 10 | 0.5 | 5 | - | - | 5 | 10 |
| butyleneglycol | 99 | 90 | 95 | 90 | 95 | 90 | - | - | - | - | - | - |
| ethylhexylglycerin | - | - | - | - | - | - | 99 | 90 | 95 | 90 | 95 | 90 |

**[Table 3]**

| Raw material (unit: wt%) | Example 28 | Example 29 | Example 30 | Example 31 | Example 32 | Example 33 |
|---|---|---|---|---|---|---|
| magnolol | 0.5 | 5 | 5 | 10 | - | - |
| honokiol | 0.5 | 5 | - | - | 5 | 10 |
| glyceryl caprylate | 99 | 90 | 95 | 90 | 95 | 90 |

**[Table 4]**

| Raw material (unit: wt%) | Comp arativ e Exam ple 1 | Comp arativ e Exam ple 2 | Comp arativ e Exam ple 3 | Comp arativ e Exam ple 4 | Com parat ive Exa mple 5 | Comp arativ e Exam ple 6 | Com parati ve Exa mple 7 | Comp arativ e Exam ple 8 | Compa rative Examp le 9 | Compa rative Examp le 10 |
|---|---|---|---|---|---|---|---|---|---|---|
| magnolol | 0.25 | 6 | 12 | - | 6 | 12 | - | 6 | 12 | - |
| honokiol | 0.25 | 6 | - | 12 | 6 | - | 12 | 3 | - | 12 |
| heptyl undecylenate | 99.5 | 88 | 88 | 88 | - | - | - | - | - | - |
| propanediol | - | - | - | - | 88 | 88 | 88 | - | - | - |
| butyleneglycol | - | - | - | - | - | - | - | 88 | 88 | 88 |

**[Table 5]**

| Raw material (unit: wt%) | Comp arativ e Exam ple 11 | Com parati ve Exam ple 12 | Comp arativ e Exam ple 13 | Com parati ve Exa mple 14 | Comp arativ e Exam ple 15 | Comp arativ e Exam ple 16 | Comp arativ e Exam ple 17 | Comp arativ e Exam ple 18 | Comp arativ e Exam ple 19 | Comp arativ e Exam ple 20 |
|---|---|---|---|---|---|---|---|---|---|---|
| magnolol | 3 | 12 | - | 3 | 12 | - | 5 | 5 | 5 | 5 |
| honokiol | 3 | - | 12 | 6 | - | 12 | 5 | 5 | 5 | 5 |
| ethylhexylglycerin | 88 | 88 | 88 | - | - | - | - | - | - | - |
| glyceryl caprylate | - | - | - | 88 | 88 | 88 | - | - | - | - |
| pentylene glycol | - | - | - | - | - | - | 90 | - | - | - |
| hexanediol | - | - | - | - | - | - | - | 90 | - | - |
| carprylyl glycol | - | - | - | - | - | - | - | - | 90 | - |
| decanediol | - | - | - | - | - | - | - | - | - | 90 |

### Experimental Example 1. Evaluation of Stability

Stability of the prepared preservatives for external skin application was evaluated.

Specifically, after storing the preservatives for external skin application according to Examples 5 to 9, 11 to 15, 17 to 21, 23 to 27, and 29 to 33, and Comparative Examples 2 to 20 in a temperature cycling chamber (2 °C↔25 °C, every 24 hours) for about 2 weeks, changes in appearance were observed.

As a result, as shown in Tables 6 to 10 and FIG. 1, in the case of the preservatives for external skin application according to the examples in which magnolol, honokiol, or a combination thereof was mixed with a solvent of heptyl undecylenate, propanediol, butyleneglycol, ethylhexylglycerin, or glyceryl caprylate, it was confirmed that significantly improved stability was obtained because a transparent, liquid state was stably maintained under refrigeration and room temperature conditions without causing solidification or precipitation. On the contrary, in the case of the preservatives for external skin application according to the comparative examples in which magnolol, honokiol, or a combination thereof was mixed with a solvent of pentylene glycol, hexanediol, carprylyl glycol, or decanediol, it was confirmed that stability significantly deteriorated because solidification or precipitation occurred under refrigeration and room temperature conditions.

In addition, as shown in Tables 6 to 10, and in FIG. 2, in the case where the weight ratio of magnolol, honokiol, or a combination thereof to heptyl undecylenate, propanediol, butyleneglycol, ethylhexylglycerin, or glyceryl caprylate, was within the range of about 1 to 10:90 to 99 according to the examples, it was confirmed that significantly improved stability was obtained because a transparent, liquid state was stably maintained under refrigeration and room temperature conditions without causing solidification or precipitation. On the contrary, in the case where the weight ratio of magnolol, honokiol, or a combination thereof to heptyl undecylenate, propanediol, butyleneglycol, ethylhexylglycerin, or glyceryl caprylate, included in the preservatives for external skin application, was out of the range of about 1 to 10:90 to 99 according to the comparative examples, it was confirmed that stability significantly deteriorated because solidification or precipitation occurred.

**[Table 6]**

| Room temperature and refrigeration conditions | Examp le 5 | Exampl e 6 | Exam ple 7 | Exam ple 8 | Exam ple 9 | Examp le 11 | Examp le 12 | Examp le 13 | Examp le 14 | Exam ple 15 |
|---|---|---|---|---|---|---|---|---|---|---|
| Refrigeration conditions (about 2 to | liquid maintai ned | liquid maintai ned | liquid maint ained | liquid maint ained | liquid maint ained | liquid maintai ned | liquid mainta ined | liquid mainta ined | liquid mainta ined | liquid maint ained |
| Room temperature conditions (about 25 °C) | liquid maintai ned | liquid maintai ned | liquid maint ained | liquid maint ained | liquid maint ained | liquid maintai ned | liquid mainta ined | liquid mainta ined | liquid mainta ined | liquid maint ained |

**[Table 7]**

| Room temperature and refrigeration conditions | Examp le 17 | Exam ple 18 | Exam ple 19 | Exam ple 20 | Examp le 21 | Exam ple 23 | Exam ple 24 | Exam ple 25 | Exam ple 26 | Examp le 27 |
|---|---|---|---|---|---|---|---|---|---|---|
| Refrigeration conditions (about 2 to 8 °C) | liquid mainta ined | liquid mainta ined | liquid mainta ined | liquid mainta ined | liquid mainta ined | liquid maint ained | liquid mainta ined | liquid mainta ined | liquid maint ained | liquid mainta ined |
| Room temperature conditions (about 25 °C) | liquid mainta ined | liquid mainta ined | liquid mainta ined | liquid mainta ined | liquid mainta ined | liquid maint ained | liquid mainta ined | liquid mainta ined | liquid maint ained | liquid mainta ined |

**[Table 8]**

| Room temperature and refrigeration conditions | Example 29 | Example 30 | Example 31 | Example 32 | Example 33 |
|---|---|---|---|---|---|
| Refrigeration conditions (about 2 to 8 °C) | liquid maintained | liquid maintained | liquid maintained | liquid maintained | liquid maintained |
| Room temperature conditions (about 25 °C) | liquid maintained | liquid maintained | liquid maintained | liquid maintained | liquid maintained |

**[Table 9]**

| Room temperature and refrigeration conditions | Compa rative Exampl e 2 | Comp arative Examp le 3 | Comp arative Exam ple 4 | Comp arative Examp le 5 | Comp arativ e Exam ple 6 | Comp arative Exam ple 7 | Compa Exampl e 8 | Comp arative Exam ple 9 | Comp arative Exam ple 10 | Compa rative Examp le 11 |
|---|---|---|---|---|---|---|---|---|---|---|
| Refrigeration conditions (about 2 to 8 °C) | precipit ation | precipi tation | precipi tation | precipi tation | precipi tation | precipi tation | precipit ation | precipi tation | precipi tation | precipit ation |
| Room temperature conditions (about 25 °C) | precipit ation | precipi tation | precipi tation | precipi tation | precipi tation | precipi tation | precipit ation | precipi tation | precipi tation | precipit ation |

**[Table 10]**

| Room temperature and refrigeration conditions | Compa rative Exampl e 12 | Comp arative Examp le 13 | Compa rative Exampl e 14 | Compa rative Exampl e 15 | Compa rative Exampl e 16 | Compa rative Exampl e 17 | Compa rative Examp le 18 | Compa rative Exampl e 19 | Compa rative Examp le 20 |
|---|---|---|---|---|---|---|---|---|---|
| Refrigeration conditions (about 2 to 8 °C) | precipit ation | precipi tation | precipit ation | precipit ation | precipit ation | precipit ation | precipit ation | solidific ation | solidifi cation |
| Room temperature conditions (about 25 °C) | precipit ation | precipi tation | precipit ation | precipit ation | precipit ation | precipit ation | precipit ation | solidific ation | solidifi cation |

Based on this experimental example, it was confirmed that the solidification or precipitation phenomenon, i.e., the issue of insolubility, of magnolol and honokiol, which may be caused when the magnolol, honokiol, or a combination thereof is mixed in a solvent, may be prevented and stability may be significantly reduced when the preservatives for external skin application include about 1 to 10 wt% of magnolol, honokiol, or a combination thereof; and about 90 to 99 wt% of heptyl undecylenate, propanediol, butyleneglycol, ethylhexylglycerin, or glyceryl caprylate.

### Experimental Example 2. Confirmation of Antibacterial Effect

Antibacterial effects of the prepared preservatives for external skin application on bacteria or fungi such as Staphylococcus aureus (ATCC 6538), Cutibacterium acnes (ATCC 6919), Candida albicans (ATCC 10231), Malassezia furfur (ATCC 14521), Trichophyton rubrum (ATCC 28188), and Aspergillus brasiliensis (ATCC 16404) were identified.

About 200 µl of a culture broth obtained by activating each strain by culturing the strain in a liquid culture medium was uniformly smeared onto a plate culture using a sterile cotton swab such that the stain was absorbed thereto. Then, a number of wells were made on the surface of the plate culture by using a sterile 7 mm-pasteur pipette, and then about 60 µl of each of the preservatives for external skin application prepared according to Examples 1 to 3, 5 to 11, 16, 17, 22, 23, 28, and 29, and Comparative Examples 1, and 17 to 20 was injected and absorbed thereto. Then, the plate culture on which Staphylococcus aureus or Malassezia furfur was smeared was incubated at about 37 °C under aerobic conditions, the plate culture on which Cutibacterium acnes was smeared was incubated at about 37 °C under anaerobic conditions, and the plate culture on which Candida albicans, Trichophyton rubrum, or Aspergillus brasiliensis was smeared was incubated at about 25 °C under aerobic conditions. After incubation for 24 to 48 hours, diameters (including the diameter (6 mm) of the well) of inhibition zones formed on the surface of each plate culture were measured. For reference, the plate culture for smearing and culturing of Staphylococcus aureus was prepared by using Tryptic Soy Agar, the plate culture for smearing and culturing of Cutibacterium acnes was prepared by using Reinforced Clostridial Agar, the plate culture for smearing and culturing of Malassezia furfur was prepared by using Leeming and Notman Agar, and the plate culture for smearing and culturing of Candida albicans, Trichophyton rubrum, or Aspergillus brasiliensis was prepared by using Potato Dextrose Agar.

As a result, as shown in Tables 11 and 12, in the case of the preservatives for external skin application according to the examples in which magnolol, honokiol, or a combination thereof was mixed with a solvent of heptyl undecylenate, propanediol, butyleneglycol, ethylhexylglycerin, or glyceryl caprylate, excellent antibacterial activity against the Gram-positive bacteria (specifically, Staphylococcus aureus and Cutibacterium acnes), yeast (specifically, Candida albicans, and Malassezia furfur), mold (specifically, Aspergillus brasiliensis and Trichophyton rubrum) was confirmed. On the contrary, in the case of the preservatives for external skin application according to the comparative examples in which magnolol, honokiol, or a combination thereof was mixed with a solvent of pentylene glycol, hexanediol, carprylyl glycol, or decanediol, significant decrease in antibacterial activity against bacteria and fungi was confirmed.

Furthermore, as shown in Tables 11 and 12, in the case of the preservatives for external skin application according to the examples in which the weight ratio of magnolol, honokiol, or a combination thereof to heptyl undecylenate, propanediol, butyleneglycol, ethylhexylglycerin, or glyceryl caprylate was within the range of about 1 to 10:90 to 99, excellent antibacterial activity against the Gram-positive bacteria (specifically, Staphylococcus aureus and Cutibacterium acnes), yeast (specifically, Candida albicans, and Malassezia furfur), mold (specifically, Aspergillus brasiliensis and Trichophyton rubrum) was confirmed. On the contrary, in the case of the preservatives for external skin application according to the comparative examples in which the weight ratio of magnolol, honokiol, or a combination thereof to heptyl undecylenate, propanediol, butyleneglycol, ethylhexylglycerin, or glyceryl caprylate was out of the range of about 1 to 10:90 to 99, significant decrease in antibacterial activity against bacteria and fungi was confirmed.

Particularly, as shown in Tables 11 and 12, it was confirmed that the antibacterial activity against bacteria and fungi was further increased in the case of including both magnolol and honokiol compared to the case of including magnolol or honokiol alone. In addition, it was confirmed that the highest antibacterial activity against bacteria and fungi was obtained in the case of the preservatives for external skin application according to Examples 5, 11, 17, 23, and 29 in which the weight ratio of magnolol: honokiol: and heptyl undecylenate, propanediol, butyleneglycol, ethylhexylglycerin, or glyceryl caprylate were about 5:5:90.

**[Table 11]**

| Raw material (unit: wt%) | Exampl e 1 | Exampl e 2 | Exampl e 3 | Exampl e 5 | Exampl e 6 | Exampl e 7 | Exampl e 8 | Exampl e 9 | Comp arativ e Exam ple 1 |
|---|---|---|---|---|---|---|---|---|---|
| magnolol | 0.5 | 1 | - | 5 | 5 | 10 | - | - | 0.25 |
| honokiol | 0.5 | - | 1 | 5 | - | - | 5 | 10 | 0.25 |
| heptyl undecylenate | 99 | 99 | 99 | 90 | 95 | 90 | 95 | 90 | 99.5 |
| Antibacterial activity test results | | | | | | | | | |
| (-: No antibacterial activity ; +: about 7 to 19 mm (inhibition zone); ++: about 20 to 30 mm (inhibition zone); +++: > about 30 mm (inhibition zone)) | | | | | | | | | |
| *S. aureus* | + | + | + | +++ | + | ++ | + | ++ | - |
| *C. acnes* | + | + | + | +++ | ++ | ++ | ++ | ++ | - |
| *C. albicans* | + | + | + | ++ | + | ++ | + | ++ | - |
| *M. furfur* | + | + | + | ++ | + | ++ | + | ++ | - |
| *A. brasiliensis* | ++ | ++ | ++ | +++ | +++ | +++ | +++ | +++ | - |
| *T. rubrum* | ++ | ++ | ++ | +++ | +++ | +++ | +++ | +++ | - |

**[Table 12]**

| Raw material (unit: wt%) | Exam ple 10 | Exam ple 11 | Exam ple 16 | Exam ple 17 | Exa mple 22 | Exam ple 23 | Exam ple 28 | Exam ple 29 | Com parat ive Exa mple 17 | Com parat ive Exa mple 18 | Com parati ve Exam ple 19 | Co mpa rativ e Exa mpl e 20 |
|---|---|---|---|---|---|---|---|---|---|---|---|---|
| magnolol | 0.5 | 5 | 0.5 | 5 | 0.5 | 5 | 0.5 | 5 | 5 | 5 | 5 | 5 |
| honokiol | 0.5 | 5 | 0.5 | 5 | 0.5 | 5 | 0.5 | 5 | 5 | 5 | 5 | 5 |
| propanediol | 99 | 90 | - | - | - | - | - | - | - | - | - | - |
| butyleneglycol | - | - | 99 | 90 | - | - | - | - | - | - | - | - |
| ethylhexylglyc erin | - | - | - | - | 99 | 90 | - | - | - | - | - | - |
| glyceryl caprylate | | | - | - | - | - | 99 | 90 | - | - | - | - |
| pentylene glycol | - | - | - | - | - | - | - | - | 90 | - | - | - |
| hexanediol | - | - | - | - | - | - | - | - | - | 90 | - | - |
| carprylyl glycol | - | - | - | - | - | - | - | - | - | - | 90 | - |
| decanediol | - | - | - | - | - | - | - | - | - | - | - | 90 |
| Antibacterial activity test results | | | | | | | | | | | | |
| (-: No antibacterial activity ; +: about 7 to 19 mm (inhibition zone); | | | | | | | | | | | | |
| ++: about 20 to 30 mm (inhibition zone); +++: > about 30 mm (inhibition zone)) | | | | | | | | | | | | |
| *S. aureus* | ++ | +++ | ++ | +++ | ++ | +++ | ++ | +++ | + | ++ | - | - |
| *C. acnes* | + | +++ | + | +++ | ++ | +++ | ++ | +++ | - | - | - | - |
| *C. albicans* | ++ | ++ | ++ | ++ | ++ | ++ | ++ | ++ | + | ++ | - | - |
| *M. furfur* | + | ++ | + | ++ | + | ++ | + | ++ | - | + | - | - |
| *A. brasiliensis* | ++ | +++ | ++ | +++ | +++ | +++ | ++ | +++ | + | ++ | - | - |
| *T. rubrum* | + | +++ | + | +++ | + | +++ | ++ | +++ | - | - | - | - |

Based on this experimental example, in the case of the preservative for external skin application including about 1 to 10 wt% of magnolol, honokiol, or a combination thereof; and about 90 to 99 wt% of heptyl undecylenate, propanediol, butyleneglycol, ethylhexylglycerin, or glyceryl caprylate, it was confirmed that excellent antibacterial effect on the Gram-positive bacteria and fungi was obtained.

Particularly, in the case of the preservatives for external skin application including about 5 wt% of magnolol; about 5 wt% of honokiol; and about 90 wt% of heptyl undecylenate, propanediol, butyleneglycol, ethylhexylglycerin, or glyceryl caprylate, the highest antibacterial effect on the Gram-positive bacteria and fungi was confirmed.

### 2. Preparation and Evaluation of Cosmetic Composition Including Preservative for External Skin Application

### Example 34 - Example 42. Preparation of Cosmetic Composition Including Preservative for External Skin Application

In these examples, cosmetic compositions including the prepared preservative for external skin application and further including cosmetic raw materials were prepared.

Specifically, one of the preservatives for external skin application of Examples 1 to 9 was mixed with cosmetic raw materials, followed by stirring to obtain a cosmetic composition in a formulation of W/O type emulsion (foundation). That is, various types of cosmetic compositions including about 1 wt% of one of the preservatives for external skin application of Examples 1 to 9, in which weight percent (wt%) of each of the other cosmetic raw materials additionally added was maintained at the same level, and the results are shown in Tables 13 and 14 below. More specifically, after sufficiently stirring Phases A and B of Tables 13 and 14 below, respectively, Phase C was added to Phase B, followed by stirring, and then Phase A was slowly added to the mixture of Phase B and Phase C while stirring. Then, Phase D was added thereto while stirrng, and then Phase E was added thereto while stirring to obtain a cosmetic composition. In addition, as shown in Tables 13 and 14, cosmetic compositions were prepared, wherein each cosmetic composition included about 1 wt% of one of the following preservatives: a preservative for external skin application including about 0.5 wt% of magnolol or honokiol and about 99.5 wt% of heptyl undecylenate; a preservative for external skin application including about 12 wt% of magnolol or honokiol and about 88 wt% of heptyl undecylenate; a preservative for external skin application including about 50 wt% of magnolol and about 50 wt% of honokiol; a preservative for external skin application including 100 wt% of magnolol; a preservative for external skin application including 100 wt% of honokiol; a preservative for external skin application including about 0.25 wt% of magnolol, about 0.25 wt% of honokiol, and about 99.5 wt% of heptyl undecylenate; a preservative for external skin application including about 6 wt% of magnolol, about 6 wt% of honokiol, and about 88 wt% of heptyl undecylenate; a preservative for external skin application including about 5 wt% of magnolol, about 5 wt% of honokiol, and about 90 wt% of pentylene glycol; and a preservative for external skin application including about 5 wt% of magnolol, 5 wt% of honokiol, and about 90 wt% of carprylyl glycol, in which weight percent (wt%) of each of the other cosmetic raw materials additionally added was maintained at the same level. These were used as controls.

**[Table 13]**

| Raw material (unit: wt%) | | Formulation example | | | | | | | | | | | | |
|---|---|---|---|---|---|---|---|---|---|---|---|---|---|---|
| | | **Exa mple 34** | **Exa mple 35** | **Exa mple 36** | **Exa mple 37** | **Exa mple 38** | **Exa mple 39** | Compa rative Examp le 21 | Compa rative Examp le 22 | Compa rative Examp le 23 | Compa rative Examp le 24 | Compa rative Examp le 25 | Compa rative Examp le 26 | Compa rative Examp le 27 |
| Phase A | purified water | Up to 100 | | | | | | | | | | | | |
| | glycerin | 3 | | | | | | | | | | | | |
| | sodium chloride | 0.9 | | | | | | | | | | | | |
| | Polvsolvate 20 | 0.9 | | | | | | | | | | | | |
| Phase B | methyl trimethicone | 23 | | | | | | | | | | | | |
| | dimethicone | 4.5 | | | | | | | | | | | | |
| | propylene glycol dicaprate | 2 | | | | | | | | | | | | |
| | PEG-10 dimethicone | 3 | | | | | | | | | | | | |
| Phase C | titanium dioxide | 10.5 | | | | | | | | | | | | |
| | polyglyceryl-2 triisostearate | 2.5 | | | | | | | | | | | | |
| Phase D | dimethicone | 8.7 | | | | | | | | | | | | |
| | silica | 4.5 | | | | | | | | | | | | |
| | **magnolol:he ptyl undecylenate =1:99 (Example 2)** | 1 | - | - | - | - | - | - | - | - | - | - | - | - |
| | **honokiol:hep tyl undecylenate =1:99 (Example 3)** | - | 1 | - | - | - | - | - | - | - | - | - | - | - |
| | **magnolol:he ptyl undecylenate =5:95 (Example 6)** | - | - | 1 | - | - | - | - | - | - | - | - | - | - |
| | **honokiol:hep tyl undecylenate =5:95 (Example 8)** | - | - | - | 1 | - | - | - | - | - | - | - | - | - |
| Phase E preser vative for extern al skin applica tion | **magnolol:he ptyl undecylenate =10:90 (Example 7)** | - | - | - | - | 1 | - | - | - | - | - | - | - | - |
| | **honokiol:hep tyl undecylenate =10:90 (Example 9)** | - | - | - | - | - | 1 | - | - | - | - | - | - | - |
| | magnolol:hept yl undecylenate =0.5:99.5 | - | - | - | - | - | - | 1 | - | - | - | - | - | - |
| | honokiol:hepty I undecylenate =0.5:99.5 | - | - | - | - | - | - | - | 1 | - | - | - | - | - |
| | magnolol:hept yl undecylenate =12:88 | - | - | - | - | - | - | - | - | 1 | - | - | - | - |
| | honokiol:hepty l undecylenate =12:88 | - | - | - | - | - | - | - | - | - | 1 | - | - | - |
| | magnolol:hon okiol=50:50 | - | - | - | - | - | - | - | - | - | - | 1 | - | - |
| | magnolol | - | - | - | - | - | - | - | - | - | - | - | 1 | - |
| | honokiol | - | - | - | - | - | - | - | - | | - | - | - | 1 |

**[Table 14]**

| Raw material (unit: wt%) | | Formulation example | | | | | | |
|---|---|---|---|---|---|---|---|---|
| | | **Exampl e 40** | **Exampl e 41** | **Exam ple 42** | Compar ative Exampl e 28 | Compa rative Exampl e 29 | Compar ative Exampl e 30 | Comp arativ e Exam ple 31 |
| Phase A | purified water | Up to 100 | | | | | | |
| | glycerin | 3 | | | | | | |
| | sodium chloride | 0.9 | | | | | | |
| | Polysolvate 20 | 0.9 | | | | | | |
| Phase B | methyl trimethicone | 23 | | | | | | |
| | dimethicone | 4.5 | | | | | | |
| | propylene glycol dicaprate | 2 | | | | | | |
| | PEG-10 dimethicone | 3 | | | | | | |
| Phase C | titanium dioxide | 10.5 | | | | | | |
| | polyglyceryl-2 triisostearate | 2.5 | | | | | | |
| Phase D | dimethicone | 8.7 | | | | | | |
| | silica | 4.5 | | | | | | |
| Phase E preservative for external skin application | **magnolol: honokiol:heptyl undecylenate=0.5:0.5:99 (Example 1)** | 1 | - | - | - | - | - | - |
| | **magnolol: honokiol:heptyl undecylenate=2.5:2.5:95 (Example 4)** | - | 1 | - | - | - | - | - |
| | **magnolol: honokiol:heptyl undecylenate=5:5:90 (Example 5)** | - | - | 1 | - | - | - | - |
| | magnolol: honokiol:heptyl undecylenate=0.25:0.25:99.5 | - | - | - | 1 | - | - | - |
| | magnolol: honokiol:heptyl undecylenate=6:6:88 | - | - | - | - | 1 | - | - |
| | magnolol: honokiol:pentylene glycol=5:5:90 | - | - | - | - | - | 1 | - |
| | magnolol: honokiol:carprylyl glycol=5:5:90 | - | - | - | - | - | - | 1 |

### Experimental Example 3. Identification of Antiseptic Activity of Cosmetic Composition Including Preservative for External Skin Application

In order to evaluate antiseptic activity of the prepared preservatives for external skin application, a certain number of bacteria was inoculated into each of the cosmetic compositions of Examples 34 to 42, which are cosmetic compositions including the preservatives for external skin application, and then viable cell count was identified. The cosmetic compositions prepared in Comparative Examples 21 to 31 were used as comparison groups.

Specifically, a culture broth of each of Cutibacterium acnes (ATCC 6919) and Candida albicans (ATCC 10231) was mixed by inoculation with each of the cosmetic compositions of Examples 34 to 42 and Comparative Examples 21 to 31, such that an initial concentration thereof per each sample was about 10⁶ CFU/g. Subsequently, while each sample was incubated at about 37 °C under anaerobic conditions (Cutibacterium acnes) or at about 25 °C under aerobic conditions (Candida albicans) for about 4 weeks, about 1 g of each sample was collected at every 7 days to measure viable cell count.

In addition, a culture broth of Aspergillus brasiliensis (ATCC 16404), which is Eumycetes, was mixed by inoculation with each of the cosmetic compositions of Examples 34 to 42 and Comparative Examples 21 to 31, such that an initial concentration thereof per each sample was about 10⁵ CFU/g. Subsequently, while each sample was incubated at about 25 °C under aerobic conditions for about 4 weeks, off-flavor and spores on the surface of the sample were observed at every 7 days.

As a result, as shown in Tables 15 and 16, in the case of the cosmetic compositions of Examples 34 to 42 including the preservatives for external skin application including magnolol, honokiol, or a combination thereof; and heptyl undecylenate in a weight ratio of about 1 to 10:90 to 99, remarkably excellent antiseptic activity against both the Gram-positive bacteria and fungi was confirmed when compared with the cosmetic compositions of Comparative Examples 21 to 31. On the contrary, in the case of comparative examples in which the weight ratio of the magnolol, honokiol, or a combination thereof to heptyl undecylenate included in the preservatives for external skin application was out of range of about 1 to 10:90 to 99, significant reduction in antiseptic activity against the Gram-positive bacteria and fungi was confirmed. In addition, in the case of the cosmetic compositions of Comparative Examples 25 to 27 including the preservatives for external skin application including only magnolol, honokiol, or a combination thereof without a solvent, significant reduction in antiseptic activity against the Gram-positive bacteria and fungi was also confirmed. Furthermore, in the case of the cosmetic compositions of Comparative Examples 30 and 31 including the preservatives for external skin application in which magnolol and honokiol were mixed by using pentylene glycol or carprylyl glycol instead of heptyl undecylenate as a solvent, significant reduction in antiseptic activity against the Gram-positive bacteria and fungi was also confirmed.

**[Table 15]**

| Cosmetic compositi on | *Cutibacterium acnes* (cfu/g) | | | | *Candida albicans* (cfu/g) | | | | *Aspergillus brasiliensis* (cfu/g) | | | |
|---|---|---|---|---|---|---|---|---|---|---|---|---|
| | Week 1 | Week 2 | Week 3 | Week 4 | Week 1 | Week 2 | Week 3 | Week 4 | Wee k 1 | Wee k 2 | Wee k 3 | Wee k 4 |
| **Example 34** | <100 | <100 | <10 | <10 | <100 | <100 | <10 | <10 | - | - | - | - |
| **Example 35** | <100 | <100 | <10 | <10 | <100 | <100 | <10 | <10 | - | - | - | - |
| **Example 36** | <10 | <10 | <10 | <10 | <10 | <10 | <10 | <10 | - | - | - | - |
| **Example 37** | <10 | <10 | <10 | <10 | <10 | <10 | <10 | <10 | - | - | - | - |
| **Example 38** | <10 | <10 | <10 | <10 | <10 | <10 | <10 | <10 | - | - | - | - |
| **Example 39** | <10 | <10 | <10 | <10 | <10 | <10 | <10 | <10 | - | - | - | - |
| Comparative Example 21 | <10³ | <100 | <100 | <100 | <10³ | <100 | <100 | <100 | - | - | - | + |
| Comparative Example 22 | <10³ | <100 | <100 | <100 | <10³ | <100 | <100 | <100 | - | - | - | + |
| Comparative Example 23 | <10⁴ | <10⁴ | <10⁴ | <10⁵ | <10⁴ | <10⁴ | <10⁴ | <10⁵ | + | + | + | ++ |
| Comparative Example 24 | <10⁴ | <10⁴ | <10⁴ | <10⁵ | <10⁴ | <10⁴ | <10⁴ | <10⁵ | + | + | + | ++ |
| Comparative Example 25 | <10⁶ | <10⁶ | <10⁶ | <10⁶ | <10⁵ | <10⁵ | <10⁵ | <10⁵ | + | + | + | ++ |
| Comparat ive Example 26 | <10⁶ | <10⁶ | <10⁷ | <10⁷ | <10⁵ | <10⁵ | <10⁵ | <10⁶ | + | + | + | ++ |
| Comparative Example 27 | <10⁶ | <10⁶ | <10⁷ | <10⁷ | <10⁵ | <10⁵ | <10⁵ | <10⁶ | + | + | + | ++ |

| | | | | | | | | | | | | |
|---|---|---|---|---|---|---|---|---|---|---|---|---|
| (-: Normal state without occurrence of off-flavor, mycelia, and spores for 4 weeks; +: occurrence of mold on some areas of the surface of the sample within 4 weeks; and ++: occurrence of off-flavor and mold over the entire surface of the sample within 4 weeks) | | | | | | | | | | | | |

**[Table 16]**

| Cosmetic compositi on | *Cutibacterium acnes* (cfu/g) | | | | *Candida albicans* (cfu/g) | | | | *Aspergillus brasiliensis* (cfu/g) | | | |
|---|---|---|---|---|---|---|---|---|---|---|---|---|
| | Week 1 | Week 2 | Week 3 | Week 4 | Wee k1 | Week 2 | Week 3 | Week 4 | Wee k 1 | Week 2 | Wee k 3 | Week 4 |
| **Example 40** | <100 | <100 | <10 | <10 | <100 | <100 | <10 | <10 | - | - | - | - |
| **Example 41** | <10 | <10 | <10 | <10 | <10 | <10 | <10 | <10 | - | - | - | - |
| **Example 42** | <10 | <10 | <10 | <10 | <10 | <10 | <10 | <10 | - | - | - | - |
| Comparative Example 28 | <10³ | <100 | <100 | <100 | <10³ | <100 | <100 | <100 | - | - | - | + |
| Comparative Example 29 | <10⁴ | <10⁴ | <10⁴ | <10⁵ | <10⁴ | <10⁴ | <10⁴ | <10⁵ | + | + | + | ++ |
| Comparative Example 30 | <10⁶ | <10⁶ | <10⁶ | <10⁶ | <10⁵ | <10⁵ | <10⁵ | <10⁵ | + | + | + | ++ |
| Comparative Example 31 | <10⁶ | <10⁶ | <10⁶ | <10⁶ | <10⁵ | <10⁵ | <10⁵ | <10⁵ | + | + | + | ++ |

| | | | | | | | | | | | | |
|---|---|---|---|---|---|---|---|---|---|---|---|---|
| (-: Normal state without occurrence of off-flavor, mycelia, and spores for 4 weeks; +: occurrence of mold on some areas of the surface of the sample within 4 weeks; and ++: occurrence of off-flavor and mold over the entire surface of the sample within 4 weeks) | | | | | | | | | | | | |

Based on the experimental example, remarkably excellent antiseptic activity against the Gram-positive bacteria and fungi was confirmed when the preservative for external skin application including about 1 to 10 wt% of magnolol, honokiol, or a combination thereof; and about 90 to 99 wt% of heptyl undecylenate was added to the cosmetic composition. Particularly, the most excellent antiseptic activity against the Gram-positive bacteria and the fungi was confirmed in the case of adding the preservative for external skin application including about 5 to 10 wt% of magnolol, honokiol, or a combination thereof; and about 90 to 95 wt% of heptyl undecylenate to the cosmetic composition.

Based on the examples and experimental examples, it was confirmed that the preservative for external skin application including magnolol, honokiol, or a combination thereof; and heptyl undecylenate, propanediol, butyleneglycol, ethylhexylglycerin, or glyceryl caprylate has superior formulation stability, excellent antibacterial and antiseptic effects, and also safety due to low skin irritation, and thus the preservative is suitable for use in cosmetic compositions.

Particularly, in the case of the preservative for external skin application including magnolol, honokiol, or a combination thereof; and heptyl undecylenate, propanediol, butyleneglycol, ethylhexylglycerin, or glyceryl caprylate in a weight ratio of about 1 to 10:90 to 99, it was confirmed that a transparent, liquid state was stably maintained without the issue of solidification or precipitation phenomenon indicating excellent formulation stability, and also excellent antibacterial and antiseptic effects not only on bacteria but also fungi, thereby solving insolubility issue of magnolol and honokiol.

Although specific embodiments of the present disclosure have been described in detail above, those skilled in the art will appreciate that these descriptions are only intended to give preferred embodiments and are not construed to limit the scope of the present disclosure. Accordingly, the substantial scope of the present disclosure will be defined by the appended claims and equivalents thereof.

## Claims

1. A preservative for external skin application, comprising:
at least one compound selected from the group consisting of magnolol and honokiol; and
at least one solvent selected from the group consisting of heptyl undecylenate, propanediol, butyleneglycol, ethylhexylglycerin, and glyceryl caprylate.

2. The preservative for external skin application of claim 1, wherein
a weight ratio of a total amount of the at least one compound selected from the group consisting of magnolol and honokiol to
a total amount of the at least one solvent selected from the group consisting of heptyl undecylenate, propanediol, butyleneglycol, ethylhexylglycerin, and glyceryl caprylate is 1 to 10:90 to 99.

3. The preservative for external skin application of claim 1, wherein
the preservative for external skin application comprises: magnolol; honokiol; and
at least one solvent selected from the group consisting of heptyl undecylenate, propanediol, butyleneglycol, ethylhexylglycerin, and glyceryl caprylate.

4. The preservative for external skin application of claim 3, wherein
a weight ratio of: the magnolol; the the honokiol;
and a total amount of the at least one solvent selected from the group consisting of heptyl undecylenate, propanediol, butyleneglycol, ethylhexylglycerin, and glyceryl caprylate is 0.5 to 5 : 0.5 to 5 : 90 to 99.

5. A cosmetic composition comprising the preservative for external skin application of any one of claims 1 to 4.

6. The cosmetic composition of claim 5, wherein
the cosmetic composition comprises 0.0001 wt% to 50 wt% of the preservative for external skin application, based on a total weight.

7. The cosmetic composition of claim 5, wherein
a formulation of the cosmetic composition is a solution, an emulsion, a suspension, a softening lotion, a nourishing lotion, a cream, a pack, a gel, a skin-adhesive cosmetic, a lipstick, a powder, a makeup base, a foundation, a shampoo, a rinse, a cleaner, an ointment, a soap, a toothpaste, a mouthwash, a paste, a lotion, a gel, a patch, a spray, or an aerosol.

8. The cosmetic composition of claim 5, wherein
the cosmetic composition further comprises an additive selected from the group consisting of purified water, carriers, emulsifiers, moisturizers, skin conditioners, surfactants, chelating agents, antioxidants, antiseptic agents, disinfectants, stabilizers, preservatives, pH regulators, lubricants, solubilizers, solvents, and combinations thereof.
